# EUROPEAN PATENT APPLICATION

(11) **EP 3 862 001 A1**
(43) Date of publication of application: **11.08.2021**
(21) Application number: 19868952.3
(22) Date of filing: 24.04.2019
(51) Int. Cl.: A61K 31/702, A61K 31/733, A61K 33/06, A61P 1/04

(54) **ANTI-HELICOBACTER AGENT AND METHOD FOR THE USE THEREOF**

(30) Priority: 04.10.2018 RU 2018134928
(71) Applicant: Chicherin, Igor Yurevich, Sergiev Posad-6, 141306 (RU)
(72) Inventor: Chicherin, Igor Yurevich, Sergiev Posad-6, 141306 (RU)
(74) Representative: Jeck, Anton
(86) International application number: PCT/RU2019/000291
(87) International publication number: WO 2020/071953

(57) **Abstract**

The group of inventions relates to the field of medicine and can be used as a medicinal agent in patients suffering from gastritis and gastric and duodenal ulcer disease caused by Helicobacter pylori (H. pylori). The technical result of the group of inventions is eliminating a toxic effect on the body from the therapeutic agents used in achieving high anti-helicobacter activity. This technical result as pertains to the agent is achieved in that the anti-helicobacter agent is a composition consisting of a prebiotic component in the form of oligofructose and inulin, and a metabolic component in the form of calcium lactate, in an effective therapeutic dose that ensures colonization resistance of the gastric mucosa and eradication of Helicobacter pylori. This technical result as pertains to the method is achieved in that the method for using the anti-helicobacter agent comprises orally administering the anti-helicobacter agent according to claims 1-5 in an effective therapeutic dose.

## Description

This group of inventions relates to the field of medicine and can be used as a medication for patients suffering from gastritis as well as gastric and duodenal ulcers caused by *Helicobacter pylori (H. pylori).*

Helicobacteriosis - is an infectious disease transmitted via a fecal-oral transmission route caused by *H. pylori* bacterium, which has a strong affinity for the epithelium of the gastric mucosa. *H. pylori* are Gram-negative, short, spiral, S-shaped bacteria of medium size, motile, with 4-5 flagella, lophotrichous, oxidase and catalase positive, exhibiting pronounced urease activity, having phosphatase, forming hydrogen sulfide, and not clotting milk. *H. pylori* urease (urea amidohydrolase) defines the main steps in *Helicobacter* pathogenesis from adhesion and gastric mucosa colonization and up to the formation of a precancerous stomach state [1-5].

As early as 1985, professor B.J. Marshall, Nobel Prize Laureate, who was one of the first to discover *H. Pylori,* while observing the positive results obtained after treatment of gastric and duodenal ulcers suggested the use of bismuth-containing triple therapy aimed at eradication of *H. Pylori.* At the same time, he pointed out that not all patients with *H. Pylori* are able to receive appropriate treatment due to their limited resources. The key factor irresponsible for ineffective eradication and recurrence of *H. Pylori* infection is antibiotic resistance.

Metronidazole and Amoxicillin, the antimicrobial drugs used as inhibitors in anti*-Helicobacter* regimen therapy, are high potency drugs causing undesirable side effects. Moreover, synthetic drugs have a toxic effect on liver. Therefore, medications with no toxic effect are proposed.

For example, the use of coniferous chlorophyll-carotene paste as an active ingredient of a pharmaceutical composition for inhibiting *Helicobacter pylori* type bacteria is known from patent No RU2367458. The disadvantage of this treatment is that the aforementioned paste was only tested *in vitro* in Petri dishes with a positive outcome achieved only at high paste concentrations of 300 mg/ml (30%). No animal *(in vivo)* or human studies have been conducted.

A pharmaceutical composition for the treatment of gastric ulcers comprising an effective amount of aluminum-containing antacid and prebiotic is known from patent No RU2315596, 2006, wherein said aluminum-containing antacid is sucralfate at a 0.25 to 1.0 g single dose or aluminum phosphate at a 3.8 to 16.0 g single dose, and said prebiotic is fructooligosaccharide (FOS) at a 2.5 to 16.0 g single dose; wherein said composition is taken 1-3 times a day. According to the study results, said pharmaceutical composition showed high efficacy in the treatment of gastric and oduodenal ulcers. However, there are no data in the patent concerning the direct anti-*Helicobacter* effect of the proposed composition.

A technical solution proposed in patent No RU2416414 is known in the art, wherein the anti- *Helicobacter* agent is calcium pectate, a non-starch polysaccharide with a low degree esterification; said polysaccharide exhibiting the following physical and chemical properties: esterification degree- 1,2%, molecular weight - 39.3 kDa, anhydrogalacturonic acid content - 67.3%, calcium - 38 mg/g per sample. Said agent was previously known as a prebiotic that prevented gastric ulcers caused by the intake of non-steroidal anti-inflammatory drugs. A significant effect of calcium pectate on *Helicobacter pylori* culture was shown *in vitro.*

The inventors of the cited patent, however, had not conducted any studies of anti- *Helicobacter* activity under clinical conditions. Various practical laboratory and clinical applications and study results indicate that efficacy of antibacterial preparations against a number of infectious agents is different under *in vitro* vs. *in vivo* conditions.

In fact, majority of antibacterial drugs against *H. Pylori* were found to be highly effective *in vitro* and ineffective *in vivo* because of the acidic environment of the stomach or their inability to penetrate the submucous membrane where the bacteria persists. (L.V. Kudryavtseva, P.L. Shcherbakov, I.O. Ivanikov , V.M. Govorun, Helicobacter pylori- infection: Aspects of Modern Diagnostics and Therapy (Physicians' Guide). Research Institute of Physical and Chemical Medicine, Ministry of Health RF, Research and Production Company "Litekh", Scientific Center for Children's Health, Central Hospital and Clinic: Clinic Administrative Healthcare Center.

The closest analog (prototype) to the claimed agent and the method of administration thereof is a technical solution disclosed in patent No 22538697. Said patent describes a pharmaceutical composition for the treatment of gastric and/or duodenal ulcers exhibiting an anti*-Helicobacter* activity and containing recombinant interferon selected from the following group: recombinant interferon-alpha, recombinant interferon-beta, recombinant interferon-gamma; antiseptics; amino acids selected from the following group: arginine, histidine, lysine, cysteine, methionine, glutamic acid; and antioxidants selected from the following group: beta-carotene, vitamin C, vitamin E, with the following component ratio in g per 1 g of the mixture:

| | |
|---|---|
| recombinant interferon, IU | 1000-10000000 |
| antiseptics | 0.00001-0.5 |
| amino acids | 0.00001-0.5 |
| antioxidants | 0.00001-0.5 |
| excipient | remaining |

According to the Claims, the composition contains an antiseptic, which can be one of the following compounds: lysozyme, biclotymol, fusafungine, ambazone, benzyldimethyl myristoylamino propylammonium, co-trimoxazole, amylmetacresol, dichlorobenzyl alcohol, cetylpyridinium chloride, and benzoxonium chloride. The disadvantage of said composition is that the antiseptics contained therein are potent drugs causing undesirable side effects. Moreover, these preparations have a toxic effect on the liver.

The background for the proposed invention was our work in the treatment of gastrointestinal tract pathologies protected by the RF patents No2593584 and No22614730. We have shown that calcium lactate restored intestinal micropinocytosis during dysbiosis, and also exhibited the anti-inflammatory activity at effective daily doses during intestinal infections by increasing the colonization resistance of the intestinal mucosa.

Thus, it can be recommended for treatment of infectious intestinal diseases both in animals and humans. However, given the specifics of *Helicobacter pylori* persistence in the gastric mucosa and the aggressive nature of the stomach contents, it was impossible to be sure that calcium lactate would be effective for the treatment of inflammatory gastric diseases caused by *Helicobacter pylor*i*.*

**The technical result of this group of inventions,** both for the medication and for the method is the elimination of toxic effects of the administered medications on the body while achieving high *anti-Helicobacter* activity.

***Said technical result for the medication*** is achieved by the preparation of the anti-*Helicobacter* agent as a composition comprising a prebiotic component in the form of oligofructose and inulin as well as a metabolic component in the form of calcium lactate, at an effective therapeutic dose that ensures colonization resistance of the gastric mucosa and eradication of *Helicobacter pylori.*

The anti*-Helicobacter* agent may contain 40-60% oligofructose, 20-30% inulin, and 15-30% calcium lactate.

The anti*-Helicobacter* agent may contain 55.1% oligofructose, 23.7% inulin, and 21.2% calcium lactate.

The anti*-Helicobacter* agent may be in the form of tablets with 0.5-1.0 g.

The anti*-Helicobacter* agent may be in the form of powder.

The anti*-Helicobacter* agent may be in the form of a solution

The effective therapeutic dose of the anti-*Helicobacter* agent for adults and children age 14 and older is 3.0-4.5 g/day.

The effective therapeutic dose of the anti*-Helicobacter* agent for children 0-3 years old is 1.0-1.5 g/day, for children 3-14 years old - 1.5-3.0 g/day.

The effective therapeutic dose of the anti*-Helicobacter* agent for animals (laboratory mice) can be 10-20 mg/day.

**Said technical result for the method** is achieved by oral administration of the anti-*Helicobacter* agent for the treatment of diseases caused by *Helicobacter pylori* at effective therapeutic doses 1-3 times a day.

The anti*-Helicobacter* agent can be administered for the treatment and prevention of gastric and duodenal ulcers caused by *Helicobacter pylori.*

The anti*-Helicobacter* agent can be administered for the treatment and prevention of gastritis caused by *Helicobacter pylori.*

The anti*-Helicobacter* agent can be administered for the treatment and prevention of experimental diseases caused by *Helicobacter pylori.*

We have studied various doses of the ingredients comprising the claimed agent. Based on the obtained results we concluded that the anti*-Helicobacter* effect is elicited by the compositions preferably comprising 40-60 g of oligofructose, 20-30 g of inulin, and 15-30 g of calcium lactate. However, the optimal therapeutic dose/response ratio, in our opinion, is the composition comprising 55.1 g of oligofructose, 23.7 g of inulin, and 21.2 g of calcium lactate in 100 g of the mixture.

### The composition is prepared as follows:

1. **Example 1.** Preparation of the claimed agent in the form of powder. The ingredients of the claimed composition are preferably taken to contain 55.1 g oligofructose, 23.7 g inulin, and 21.2 g calcium lactate in 100 g of the composition and mixed together.
2. **Example 2.** Preparation of the claimed agent in the form of tablets. The ingredients of the claimed composition are preferably taken to contain 55.1 g oligofructose, 23.7 g inulin, and 21.2 g calcium lactate in 100 g of the composition and tableted by a known method to obtain tablets weighing 0.5-1.0 g.
3. **Example 3.** Preparation of the claimed agent in the form of a solution. The ingredients of the claimed group are preferably taken to contain 55.1 g oligofructose, 23.7 g inulin, and 21.2 g calcium lactate in 100 g of the composition, mixed together, and dissolved before use in boiled or distilled water to prepare the aforementioned effective therapeutic daily dose for children age 0-3 to be contained in 15 ml of the liquid (5 ml, 3 times a day), for children age 3-14 to be contained in 60 ml (20 ml, 3 times a day), and for adults and children age 14 and older to be contained in 90 ml (30 ml 3 times a day).

### The method is carried out as follows:

Because the claimed agent contains prebiotics:
Fructooligosaccharide
+ Fructopolysaccharide and also, calcium lactate (metabiotic), the inventors classified the claimed agent as metaprebiotic.

The agent prepared by said method in any of the three forms described above is administered orally.

The effective therapeutic dose for adults and children age 14 and older can be 3.0-4.5 g/day, for children 0-3 years old this dose can be 1.0-1.5 g/day, for children 3-14 years of age - 1.5-3.0 g/day.

When administering the claimed anti*-Helicobacter* agent to laboratory animals, such as mice, the effective therapeutic dose can be 10-20 mg/day. The preparation is administered into the stomach preferably as a solution through a G-tube.

### Study of H. Pylori bacteria sensitivity to the claimed agent, the fructooligosaccharide+fructopolysaccharide complex

### (FOS+FPS), Ca Lactate

*H. Pylori* culture was inoculated on a solid growth medium and incubated at 37°C under microaerophilic conditions. The dishes were covered with discs impregnated with Cefotaxime and Pefloxacin antibiotics and coarse calico test sheets impregnated with the claimed agent solutions (100 g of the composition contained 55.1 g oligofructose, 23.7 g Inulin, and 21.2 g calcium lactate), Ca Lactate (concentration: 2 mg in 1 ml distilled water).

The photograph shows the growing *H. Pylori* culture and growth inhibition zones formed around the discs and test sheets.

Location of the antibacterial agents on the surface of the solid growth medium in a Petri dish: top left -metaprebiotic as the claimed agent (100 g of the composition contained 55.1 g oligofructose, 23.7 g Inulin, and 21.2 g calcium lactate), top right - FOS+FPS complex, bottom right - Ca Lactate, bottom left -pefloxacin, center - Cefotaxime.

The proposed metaprebiotic exhibits both bacteriostatic and bactericidal activity (diameter of the growth inhibition zone of *H. Pylori* bacteria is up to 12 mm); for the FOS-FPS complex, the growth inhibition zone diameter is smaller, up to 10 mm, the bacteriostatic activity zone is somewhat lower; in the *in vitro* experiment, Ca lactate exhibits the bacteriostatic effect only.

### Experimental verification of anti-Helicobacter activity of the claimed agent

To study various aspects of the interaction between *H. Pylori* 11 bacterial strain and the bodies of white mice, *H. pylori* KM-11 (Rif11) strain labelled for resistance to Rifampicin, which can be used for its differentiation with other types of microorganisms, was prepared by spontaneous mutagenesis described in Handbook [6,7].

Helicobacteriosis was induced in white mice by oral administration of *H. pylori* KM-11 (Rif11) bacterial suspension for 5 days to the animals who had been intramuscularly administered Dexamethasone (manufactured by KRKA, Slovenia) at a 40 mcg per animal daily dose to induce the immunosuppressive effect. Invention application No 2018117831 for said method for simulating said pathology was filed on 05.14.2018, see also I.Yu. Chicherin, I.P.Pogorelsky, I. A. Lundovskikh, A.S. Gorshkov, M.R. Shabalina, D.N. Smirnova, N.V. Bogacheva Experimental Helicobacteriosis in Laboratory White Mice Infected with Helicobacter pylori Pathogen. Infectious diseases. 2008; 16(2): 77-85.

Dexamethasone induces the immunosuppressive effect by involuting lymphoid tissue, suppressing proliferation of lymphocytes (especially T-cells), inhibiting B-cell migration and T- and B-cell interaction, repressing cytokine migration (interleukin 1, interleukin 2, interferon gamma) from lymphocytes and macrophages, and reducing antibody formation.

*H. pylori* KM-11 (Rif^{R}) bacteria isolated from the feces of animals is an indicator of *Helicobacter's* reproduction in the stomach followed by its penetration into the intestine.

*H. pylori* KM-11 (Rif^{R}) bacteria was cultured under microaerophilic conditions at 37°C for 24 hrs. after which a bacterial suspension was prepared in an isotonic sodium chloride solution at a dose of 2.10 microbial cells per 0.2 ml.

After Dexamethasone had been administered (on the second day after the start of administration), the animals were orally administered 0.2 ml *H. pylori* KM-11 (Rif^{R}) bacterial suspension in isotonic sodium chloride solution once a day for 4 days, collecting animal feces daily to determine the amount of *Helicobacter* excreted from the animals.

Upon completion of the administration of *H. pylori* KM-11 (Rif^{R}) bacterial suspension, the animals were divided into four treatment groups: Group 1: treatment with the claimed agent (100 g of the composition contained 55.1 g oligofructose, 23.7 g inulin, and 21.2 g calcium lactate), Group 2: treatment with FOS-FPS; Group 3: treatment with Ca lactate; Group 4: no treatment (control group of animals). From the beginning of treatment, the contents of the animal stomachs were collected for homogenization and determination of the *Helicobacter* content.

The results obtained from culturing intestinal homogenate suspensions and fecal suspensions of white mice on a selective medium with Rifampicin were processed based on the plate count after which a decision was made on the colonization rate of *H. pylori* KM-11 (Rif^{R}) bacteria in the intestinal wall, the start of *H. pylori* KM-11 (Rif^{R}) bacterial excretion with animal feces, intensity and duration of *H. pylori* KM-11 (Rif^{R}) bacterial excretion.

Based on said experimental results, the following conclusion can be made: *H*. *pylori* appear in feces of experimental animals (375 KOE/1 g) on the third day post intramuscular administration of Dexamethasone and on the second day (also post intramuscular administration of Dexamethasone) post oral administration of the bacteria, and later isolated even from the feces of the animals untreated up to the end of the experiment, gradually decreasing in numbers to 13 KOE/1 g at the end of the experiment (observation period).

Thus, the duration of *Helicobacter* bacterial excretion with feces from 01.30.2018 to 02.12.2018 (as per the results in the animal group in which no agents with metaprebiotic action were administered) was 14 days. Treating animals with the preparations reduced the amount of bacteria excreted with feces to 2-6 KOE/1 g. Post administration of the preparation with the metaprebiotic action to the animals, the amount of *H. pylori* in feces continued to drop 5 more days.

The duration of *Helicobacter* bacterial excretion with feces from 02.05.2018 to 02.12.2018 (as per the results in the animal group in which no preparations with metaprebiotic action were administered) was 8 days.

On Day 5 of intramuscular Dexamethasone administration (and Day 4 of oral administration of *H. pylori* to the animals), 2.6 million bacteria per 1 g of the organ tissue were detected in gastric homogenates, which indicated their adhesion and colonization of the gastric mucosa.

### When administering medications with metaprebiotic action for 4 days, total clearance of H. pylori bacteria from the body occurred by the 13^{th} day, i.e. from 01.31.2018 to 02.13.2018.

### Verification of anti-Helicobacter activity of the claimed agent under clinical conditions

The study was conducted on male volunteers, 27 to 69 year of age. *H. pylori* KM-11 (Rif^{R}) bacterial culture was plated on solid growth medium with Rifampicin under microaerophilic conditions at 37°C for 24 hrs.; the bacteria were then suspended in isotonic sodium chloride solution at 10 billion microbial cells in a volume of 30 ml.

An important indicator of *Helicobacter's* reproduction in the stomach and its further penetration into the intestine is isolation of *H. pylori* KM-11 (Rif^{R}) from the feces of volunteers.

On Day 3 of the experiment, (i.e. one day after *H. pylori* KM-11 (Rif^{R}) suspension was administered), the feces of all volunteers showed Rif^{R}- labelled *Helicobacter* bacteria (See Table). The data in the table demonstrate that on Day 5 of the experiment, *H. pylori* KM-11 (Rif^{R}) bacterial content in the feces of the volunteers reached the highest level and remained essentially at the same level up to Day 7 of the experiment, after which *H. pylori* KM-11 (Rif^{R}) bacterial suspension was no longer administered to the volunteers.

The high content of *H. pylori* KM-11 (Rif^{R}) bacteria in the feces of volunteers persisted even on Day 7, the first day of receiving the metabiotic in the form of the claimed agent (100 g of the composition contained 55.1 g oligofructose, 23.7 g Inulin, and 21.2 g calcium lactate), which was followed by a sharp decline in the amount of *Helicobacter* bacteria in the feces up to Day 11 of the experiment (Day 5 of the metabiotic administration). These results are presented in Table "Results of determining the amount of *H. pylori* KM-11 (Rif^{R}) bacteria in the feces of volunteers during the entire observation period."

On Day 12 of the experiment all volunteers had a fairly significant increase in the amount of *H. pylori* KM-11 (Rif^{R}) bacteria in the fecal samples taken for bacteriological study. This increase in the amount of *Helicobacter* in the feces of the volunteers who received a metabiotic in the form of the claimed agent can probably be attributed to the "secretion" into the stomach lumen of *H. pylori* KM-11 (Rif^{R}) microbial cells that were spilled over due to the increased colonization resistance of the gastric mucosa and activation of the indigenous microbiota triggered by the metaprebiotic.

This hypothesis is viable because when volunteers continued receiving the claimed metaprebiotic, the amount of *Helicobacter* in the feces sharply decreased until their complete elimination by Day 17 of the experiment (by Day 11 of receiving the metaprebiotic). In fact, complete eradication of the Helicobacteriosis pathogen resulting from the metaprebiotic therapy was observed.

On Days 8 and 9 of the experiment, three volunteers reported heartburn, which ceased one day later after taking the metaprebiotic. On Day 12 of the experiment the unpleasant sensation in the epigastric region disappeared. On a separate note, on Day 12-14 of the experiment, four of the volunteers reported increased stool frequency and changes in the fecal color; the feces acquired a reddish-brown color, same as the supernatant liquid after centrifugation of fecal samples, which was regarded as a manifestation of acute gastritis. The short-term change in the fecal color and stooling frequency ceased in the following days of the experiment while the volunteers continued to receive the metaprebiotic in the form of the claimed agent.

At the end of the two-week period of receiving said metaprebiotic and later (in 2 weeks and one month), a bacteriological study of the feces of the volunteers didn't reveal any *H. pylori* KM-11 (Rif^{R}) microbial cells. No changes in the wellbeing of the volunteers and their work performance were observed thereafter.

The presented positive results give grounds for certain optimism. Both in the case of the experimental animals whose stomachs were proven to be completely clear of *Helicobacter* pathogens during administration of the medication with a prebiotic action - the claimed agent, a composition comprising a prebiotic component in the form of oligofructose and inulin as well as a metabolic component in the form of calcium lactate at an effective therapeutic dose, FOS+FPS, Ca Lactate, and in the course of the experimental study where volunteers were self-infected, we are getting close to understanding that the dream of clinicians can be realized.

In both cases, in the experiments on laboratory white mice and in the experiments on self-infected volunteers, the therapeutic efficacy of the claimed agent providing colonization resistance of the gastric mucosa and eradication of *Helicobacter pylori* was tested. This preparation can be considered quite close to an "ideal antimicrobial agent" based on the following therapeutic effects thereof:
- not a classic antibiotic and no resistance to it can be developed;
- narrow-spectrum agent. Inhibits the growth of *H. pylori* while stimulating the growth of indigenous microbiota (experimentally-supported data);
- characteristically stable antagonistic activity against *H. pylori* in the acidic environment of the stomach in both experimental animals and human volunteers;
- ability to penetrate the mucosa, reach the target on the surface of *H. pylori* without losing its antimicrobial activity and also improve the colonization resistance of gastric mucosa and promote its decontamination by *H. pylori.*

**Table**

| Day of the experiment | Suspension *H. pylori* KM-11 (Rif^{R}) administered | Metabiotic in the form of the claimed agent administered | Content of *H. pylori* KM-11 (Rif^{R}) in feces of volunteers, KOE (r^{-1,}(x± I₉₅, n=5) | | | | |
|---|---|---|---|---|---|---|---|
| | | | 1 | 2 | 3 | 4 | 5 |
| 1 | - | - | 0 | 0 | 0 | 0 | 0 |
| 2 | + | - | 0 | 0 | 0 | 0 | 0 |
| 3 | + | - | 96±8 | 82±7 | 80±9 | 74±8 | 65±37 |
| 4 | + | - | 970±34 | 1112±86 | 1070±78 | 996±42 | 870±37 |
| 5 | + | - | (9.9±0.6) 10⁵ | (1.3±0.7) 10⁶ | (1.3±0.7) 10⁶ | (1.0±0.6) 10⁶ | (1.3±0.5) 10⁶ |
| 6 | + | - | (9.9±0.5) 10⁵ | (9.9±0.7) 10⁵ | (9.9±0.6) 10⁵ | (8.9±0.7) 10⁵ | (9.0±0.6) 10⁵ |
| 7 | - | + | (5.1 ±0.7) 10⁵ | (5.7±0.6) 10⁵ | (4.9±0.8) 10⁵ | (4.8±0.6) 10⁵ | (6.9±0.8) 10⁵ |
| 8 | - | + | 310±34 | 375±42 | 506±68 | 498±58 | 458±48 |
| 9 | - | + | 360±38 | 410±36 | 390±39 | 386±42 | 356±43 |
| 10 | - | + | 160±38 | 56±8 | 10±3 | 280±29 | 120±26 |
| 11 | - | + | 99±8 | 114±29 | 200±31 | 195±29 | 206±33 |
| 12 | - | + | (1.3±0.6) 10⁵ | (1.0±0.8) 10⁵ | (2.0±0.6) 10⁵ | (1.9±0.8) 10⁵ | (1.7±0.7) 10⁵ |
| 13 | - | + | 420±42 | 510±58 | 1280±66 | 630±47 | 720±62 |
| 14 | - | + | 82±8 | 109±28 | 320±27 | 89±9 | 68±9 |
| 15 | - | + | 0 | 0 | 39±7 | 0 | 0 |
| 16 | - | + | 0 | 0 | 2 | 0 | 0 |
| 17 | - | + | 0 | 0 | 0 | 0 | 0 |
| 18 | - | + | 0 | 0 | 0 | 0 | 0 |
| 19 | - | + | 0 | 0 | 0 | 0 | 0 |
| 20 | - | + | 0 | 0 | 0 | 0 | 0 |
| 21-25 | - | - | 0 | 0 | 0 | 0 | 0 |
| 2 weeks after completion of treatment | - | - | 0 | 0 | 0 | 0 | 0 |
| 1 month after completion of treatment | - | - | 0 | 0 | 0 | 0 | 0 |

### List of the cited literature

1. Morris F., Maher K., Thomsen L. et al. Distribution of Campylobacter pylori in the human stomach obtained at postmortem // Scand. J. Gastroenterol. - 1988. - Vol. 23. - pp. 257-264.
2. Tsujii M., Kawano S., Tsujii S. et al. Ammonia: a possible promotor in Helicobacter pylori - related gastric carcinogenesis // Cancer Lett. - 1992. -Vol. 65.-pp.15-18.
3. Nizhevich A.A., Khasanov R.Sh. Helicobacter pylori: introduction into pathogenesis and pathobiochemistry of gastritis// Proceedings of the VIII Thematic Symposium of the Russian Helicobacter pylori Study Group, May 18, 1999, Ufa. / www.Helicobacter.ru/index. php?i=59
4. Helicobacter pylori-infection: Modern Diagnostic and Therapy (Physicians' Guide)/ Scientific Research Institute of Physical and Chemical Medicine, Ministry of Health, RF; Research and Production Company "Litekh", Scientific Center for Children's Health, Central Hospital and Clinic: Clinic Administrative Healthcare Center, Office of the President of the RF.- M. - 2004. - 41 pages.
5. Tsujii M., Kawano S., Tsujii S. et al. Cell kinetics of mucosal atrophy in rat stomach induced by long-term administration of ammonia // Gastroenterology. -1933. -Vol. - 104.- P.769-801.; Tsujii M., Kawano S., Tsujii S. et al. Mechanism for ammonia-induced promotor of gastric carcinogenesis in rats // Carcinogenesis. -1995. - Vol. 16. - pp. 563-566).
6. Miller G. Experiments in Molecular Genetics / Translated from the English by Yu.N. Zograf, T.S Ilyina, V.G. Nikiforova. - M.: Mir, 1976. - 436 pages.
7. I.V. Darmov, I.Yu. Chicherin, I.P. Pogorelsky, I.Ya. Lundovskikh, S.N. Yanov. Method for the Evaluation of Survival of Bifidobacteria and Lactobacillus in the Gastrointestinal Tract of Experimental Animals. Patent No 2528867. Russian Federation, publ. 09.20.2014. Bul. No 26. UPC No: C12Q 1/04, UPC C12Q 1/06.

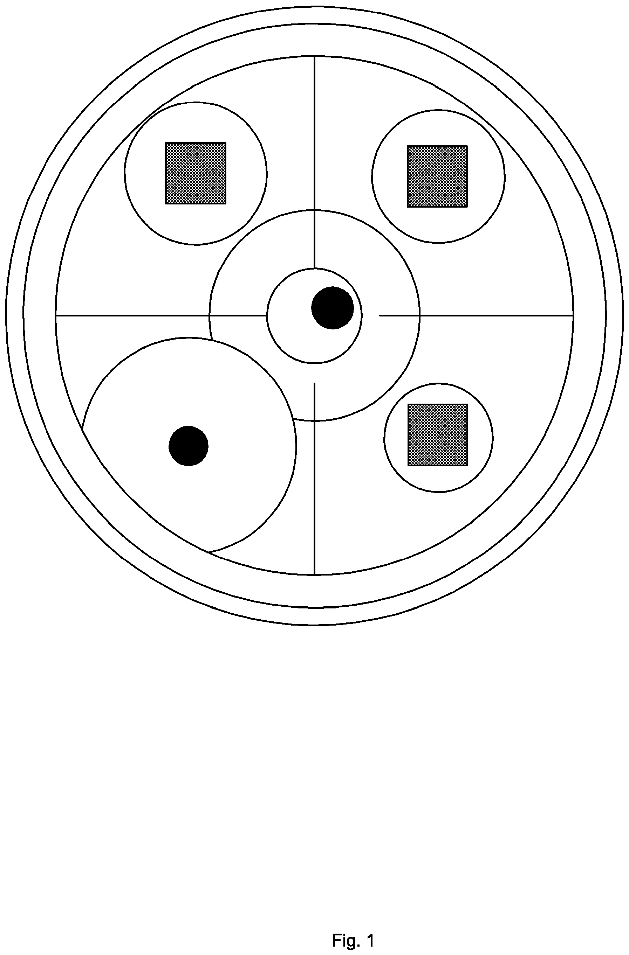

## Claims

1. An anti*-Helicobacter* agent, wherein said agent is a composition comprising a prebiotic component in the form of oligofructose and inulin as well as a metabolic component in the form of calcium lactate, at an effective therapeutic dose that provides colonization resistance of the gastric mucosa and eradication of *Helicobacter pylor*i*.*

2. The anti*-Helicobacter* agent according to claim 1, wherein said agent contains 40-60 % oligofructose, 20-30 % inulin, and 15-30 % calcium lactate.

3. The anti*-Helicobacter* agent according to claim 1, wherein said agent contains 55.1 % oligofructose, 23.7 % inulin, and 21.2 % calcium lactate.

4. The anti*-Helicobacter* agent according to claim 1, wherein said agent is in the form of tablets 0.5-1.0 g each.

5. The anti*-Helicobacter* agent according to claim 1, wherein said agent is in the form of powder.

6. The anti*-Helicobacter* agent according to claim 1, wherein said agent is in the form of a solution.

7. The anti*-Helicobacter* agent according to claim 1, wherein the effective therapeutic dose for adults and children 14 years and older is 3.0-4.5 g per day.

8. The anti*-Helicobacter* agent according to claim 1, wherein the effective therapeutic dose for children 1 to 3 years old is 1.0 - 1.5 g per day, and for children 3 to 14 years old is 1.5-3.0 per day.

9. The anti*-Helicobacter* agent according to claim 1, wherein the effective therapeutic dose for animals, laboratory mice, is 10 - 20 mg/day.

10. A method for using the anti*-Helicobacter* agent according to claims 1-9, wherein said agent is administered orally at the effective therapeutic dose 1-3 times per day.

11. The method for using the anti*-Helicobacter* agent according to claim 10, wherein said agent is administered for the treatment of conditions caused by *Helicobacter pylori.*

12. The method for using the anti*-Helicobacter* agent according to claim 10, wherein the *anti-Helicobacter* agent is administered for the treatment and prevention of gastric and duodenal ulcers caused by *Helicobacter pylori.*

13. The method for using the anti-*Helicobacter* agent according to claim 10, wherein the anti*-Helicobacter* agent is administered for the treatment and prevention of gastritis caused by *Helicobacter pylori.*

14. The method for using the anti*-Helicobacter* agent according to claim 10, wherein the anti*-Helicobacter* agent is administered for the treatment and prevention of experimental diseases caused by *Helicobacter pylori.*
